# EUROPÄISCHE PATENTSCHRIFT

(11) **EP 3 964 248 B1**
(45) Veröffentlichungstag und Bekanntmachung des Hinweises auf die Patenterteilung: **13.11.2024**
(21) Anmeldenummer: 21195365.8
(22) Anmeldetag: 07.09.2021
(51) Int. Cl.: A61M 5/142, A61M 39/08

(54) **SPRITZGUSS-SCHLAUCH**
INJECTION MOLDED HOSE
TUYAU MOULÉ PAR INJECTION

(30) Priorität: 08.09.2020 CH 11132020
(43) Veröffentlichungstag der Anmeldung: 09.03.2022
(73) Patentinhaber: INTEGRA Biosciences AG, 7205 Zizers (CH)
(72) Erfinder: Shearer, Daniel, 7415 Pratval (CH); Pasquier, Noel, 7302 Landquart (CH); Städler, Andreas, 7012 Felsberg (CH)
(74) Vertreter: Swisspat Riederer Hasler Patentanwälte AG

(56) Entgegenhaltungen:
- EP-A1- 2 754 462
- EP-A1- 3 103 614
- WO-A1-00/57941
- US-A1- 2012 034 117

## Beschreibung

### TECHNISCHES GEBIET DER ERFINDUNG

Die Erfindung betrifft einen Schlauch, insbesondere zum Anbringen in einer Dispensier-Kassette, gemäss Oberbegriff des Anspruchs 1. Des Weiteren betrifft die Erfindung eine Schlauchverbindung mit einem solchen Schlauch, ein Verfahren zur Herstellung und eine Verwendung eines solchen Schlauchs.

### HINTERGRUND DER ERFINDUNG

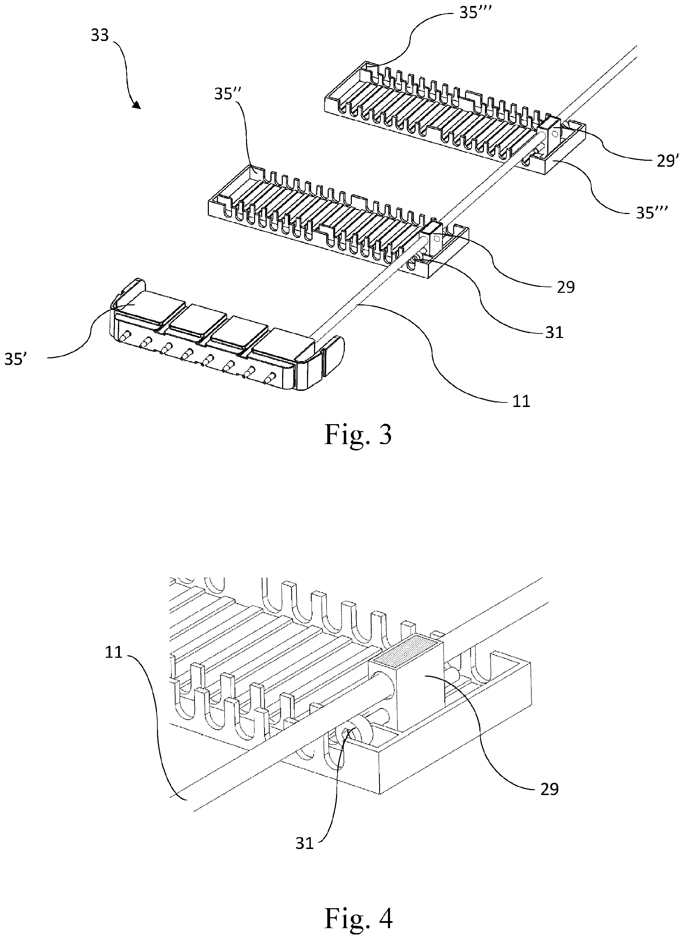

Die aus dem Stand der Technik bekannten Dispensierkassetten zur Anwendung in einer Peristaltikpumpe weisen einen mehrteiligen Aufbau auf. Die Dispensier-Kassette umfasst zwei bis drei Kartuschen sowie Schläuche, welche jeweils von der ersten Kartusche angefangen über die zweite Kartusche bis zu einer möglichen dritten Kartusche führen (siehe Figur 3). Die Anzahl der Schläuche in einer Dispensierkassette, welche nebeneinander in einer Reihe und parallel zueinander angeordnet sind, beträgt in der Regel maximal 16. Die Schläuche zwischen der ersten und zweiten bzw. zwischen der zweiten und dritten Kartusche kommen in einer Peristaltikpumpe auf deren Rotor zu liegen. Aufgrund der Tatsache, dass die auf dem Rotor angeordneten Schläuche grösseren Beanspruchungen ausgesetzt sind, können diese Schläuche separat zur Verfügung gestellt sein und brauchen nicht die ganze Länge von der ersten bis zur letzten Kartusche abzudecken. In der ersten Kartusche sind nebst den Schläuchen auch Dispensier-Spitzen angebracht, welche das in den Schläuchen transportierte Medium nach aussen fördern. In der dritten Kartusche sind Konnektoren vorgesehen, die als Schnittstelle zum Behälter, in welchem das zu dispensierende Medium aufbewahrt wird, dienen. Bei einer aus dem Stand der Technik bekannten Dispensier-Kassette muss jede Kartusche einzeln an der Peristaltikpumpe angebracht werden. Bei einem dreiteiligen Aufbau der Dispensier-Kassette führt dies zu drei nacheinander durchzuführenden Arbeitsschritten, welche sowohl beim Anbringen an als auch beim Lösen von der Peristaltikpumpe vorzunehmen sind.

Die in einer Dispensier-Kassette eingesetzten Schläuche müssen eine flexible Wand aufweisen. Das Befördern des Mediums in den Schläuchen geschieht durch die Verwendung der Peristaltik. Das heisst, dass aufgrund der Verformung des Schlauchs das Medium innerhalb des Schlauchs in eine Richtung gezielt gefördert wird. Die Schläuche in der Dispensier-Kassette müssen mit einer bestimmten Spannung auf dem Rotor der Peristaltikpumpe gespannt sein, um überhaupt die Verformung der Schläuche durch den Rotor und somit auch eine Dispensierung eines Mediums zu ermöglichen.

Die Schläuche für die Verwendung in Dispensier-Kassetten werden durch Extrudieren hergestellt. Dieses Verfahren weist den Nachteil auf, dass die Toleranz des Durchmessers des Schlauchs und somit die Unterschiede der Innendurchmesser zwischen den Schläuchen relativ gross sind. Dies führt wiederum unter gleichen Randbedingungen zu unterschiedlichen Fördermengen zwischen den Schläuchen. Der Ausgleich der Fördermenge unter den Schläuchen wird durch Verändern der Schlauchlänge erzielt. Die Veränderung der Länge bewirkt eine Änderung des Querschnitts und zugleich des Volumens, wobei der Querschnitt wiederum einen direkten Einfluss auf die Fördermenge des einzelnen Schlauchs aufweist. Die Länge und somit auch der Durchmesser jedes einzelnen Schlauchs kann mit Hilfe der am Schlauch angelegten Spannung eingestellt werden, um die identische Fördermenge unter den Schläuchen zu erreichen. Aus diesem Grund sind an den zurzeit gängigen Kartuschen Spannelemente für jeden einzelnen Schlauch vorgesehen. Mit Hilfe einer Stellschraube an diesen Spannelementen lassen sich die am Schlauch angelegte Spannung und gleichzeitig die Länge und der Durchmesser des Schlauchs einstellen.

Eine der Ursachen für die Erfordernis, die Spannung über jeden Schlauch einzeln einzustellen, ist der bereits oben erwähnte unterschiedliche Durchmesser der Schläuche. Das zurzeit gängige Verfahren zur Herstellung von Schläuchen für die Verwendung in Dispensier-Kassetten ist das Extrudieren. Dieses Verfahren weist den Nachteil auf, dass bei einer Herstellung eines Schlauchs der Durchmesser des Schlauchs von der Herstell-Geschwindigkeit abhängt. Das heisst, dass bereits leichte Veränderungen in der Geschwindigkeit des Extrusion-Erzeugnisses zu einem unterschiedlichen Schlauch-Durchmesser führen.

Sowohl das Einstellen der Schlauchspannung für jeden einzelnen Schlauch als auch das schrittweise Anbringen der Kartuschen führen zu einem erheblichen Aufwand bei der Installation der Dispensierkassette an einer Peristaltikpumpe. Dieser Zeitaufwand überträgt sich direkt auf die Produktionszeit, was sich wiederum in einem ineffizienten Produktionsprozess niederschlägt.

Die Fördermenge durch einen Schlauch ist von seinem Durchmesser und somit unter anderem von der an ihm angelegten Spannung abhängig. Da der Durchmesser der Schläuche aufgrund der Herstellungsmethode variiert, muss die Spannung dementsprechend für jeden Schlauch individuell eingestellt werden. Einzig auf diese Weise kann erreicht werden, dass die Vielzahl von eingesetzten Schläuchen die identische Fördermenge aufweist.

In WO 00/57941 ist ein Schlauch zur Verwendung in einer Infusionspumpe gezeigt, welcher in einem Spritzguss-Verfahren, bevorzugt aus Silikon, hergestellt ist. Der Schlauch weist einen ersten Abschnitt auf, welcher auf dem Rotor der Infusionspumpe zu liegen kommt. Durch Drehen des Rotors wird der Schlauch auf das gegenüberliegend angebrachte Schlauchbett der Infusionspumpe gedrückt und somit der Schlauch abgeklemmt. Ein zweiter Abschnitt des Schlauchs weist eine in Bezug zum ersten Abschnitt unterschiedliche Oberflächen-Struktur der Innenfläche auf. Diese unterschiedliche Struktur der Innenseite des zweiten Abschnitts ermöglicht das Durchführen von optischen Messungen während der Verwendung des Schlauchs in einer Infusionspumpe.

Auf der Aussenfläche des Schlauchs können Absätze angeformt sein, welche zur Positionierung des Schlauchs in einer Infusionspumpe dienen. Der Aussendurchmesser des Schlauchs beträgt ungefähr 0.4 cm, was zu einer Aussenquerschnittsfläche von etwa 12.5 mm² führt. Der Schlauch wird an seinen beiden Enden an der Infusionspumpe mit Hilfe einer Klemmeinrichtung angebracht und gehalten.

### AUFGABE

Es ist deshalb eine Aufgabe der vorliegenden Erfindung, einen Schlauch vorzuschlagen, welcher die gewünschte Fördermenge aufweist, ohne eine Einstellung am Schlauch vornehmen zu müssen. Zugleich soll ein Schlauch gezeigt werden, welcher in einer Peristaltikpumpe ohne Schlauchbett verwendet werden kann und eine grosse Haltbarkeit bzw. Lebensdauer aufweist.

Des Weiteren gilt es eine Schlauchverbindung vorzuschlagen, welche ebenfalls eine gewünschte Fördermenge gewährleistet, ohne eine Einstellung an irgendeinem Schlauchstück der Schlauchverbindung vornehmen zu müssen. Eine weitere Aufgabe der vorliegenden Erfindung ist das Aufzeigen eines Verfahrens zur Herstellung und einer Verwendung eines solchen Schlauchs.

### BESCHREIBUNG

Die oben genannten Aufgaben werden erfindungsgemäss gelöst durch einen Schlauch nach Anspruch 1, welcher sich dadurch auszeichnet, dass er in einem Spritzgussverfahren hergestellt ist und die Toleranz seiner Innen-Querschnittsfläche maximal ±33% beträgt, eine Schlauchverbindung nach Anspruch 11 und ein Verfahren zur Herstellung eines Schlauchs nach Anspruch 16. Vorteilhafte Ausgestaltungen des Schlauchs werden in den abhängigen Ansprüchen umschrieben.

Die Erfindung bezieht sich auf einen Schlauch zum Anbringen an einer Dispensier-Kassette zur Aufnahme in einer Peristaltikpumpe und zum Spannen über einen Rotor der Peristaltikpumpe und Fördern eines Mediums durch äussere mechanische Verformung des Schlauchs. Der Schlauch weist eine Länge von vorzugsweise 20 mm bis 200 mm, eine Innen-Querschnittsfläche von 0.07 bis 7.07 mm² und eine Wandstärke von 0.3 mm bis 2.2, mm, vorzugsweise von 0.3 bis 1.5 mm, auf. An den Enden des Schlauchs sind Verdickungen angeformt und die Distanz zwischen den Verdickungen beträgt zwischen 40 und 100 mm. Der Schlauch ist erfindungsgemäss in einem Spritzgussverfahren hergestellt und geeignet, um beim Spannen über den Rotor mit einer Spannkraft von 1 bis 10 N vollständig zu schliessen. Die Innen-Querschnittsfläche des Schlauchs weist eine Toleranz von maximal ± 33% auf.

Durch das Spritzgussverfahren wird ein einstückiger Schlauch hergestellt. Damit sind die Verdickungen aufgrund des Herstellungsverfahrens stoffschlüssig mit dem Schlauch verbunden. Dies ermöglicht in einem einzigen Verfahrensschritt einen Schlauch zu produzieren, welcher Verdickungen an seinen Enden aufweist. Eine nachträgliche Nachbearbeitung ist nicht notwendig. Das Herstellungsverfahren des Spritzgiessens bringt beim vorliegenden Erfindungsgegenstand den Vorteil mit sich, dass die Toleranzen der Dimensionen eines derart hergestellten Schlauchs im Vergleich zu anderen Herstellungsverfahren sehr klein sind. Beim Schlauch zum Anbringen an einer Dispensier-Kassette zur Aufnahme in einer Peristaltikpumpe hat die kleine Toleranz einen grossen Einfluss auf den Einsatz des Schlauchs. Denn ein Schlauch in einer Peristaltikpumpe ist vorgesehen eine bestimmte Menge von Flüssigkeit zu dispensieren. Dabei sollte diese Menge so konstant wie möglich sein, und zwar nicht nur über die Zeit sondern auch unter den Schläuchen. Auf die Menge an Flüssigkeit, welche durch einen Schlauch in einer Peristaltikpumpe gefördert wird, hat die Innen-Querschnittsfläche des Schlauchs einen direkten Einfluss. Die Änderung der Innen-Querschnittsfläche von Schläuchen untereinander führt zu entsprechend unterschiedlichen Fördermengen durch diese Schläuche. Im Stand der Technik muss der Unterschied der Innen-Querschnittsflächen von Schläuchen durch Anpassung der Spannung der Schläuche kompensiert werden. Dies erfordert die individuelle Einstellung der Spannung für jeden Schlauch. Durch eine kleine Toleranz der Innen-Querschnittsfläche kann auf eine individuelle Einstellung der Schlauch-Spannung verzichtet werden, was die Verwendung einer Dispensier-Kassette mit solchen Schläuchen erheblich erleichtert. Aufgrund des Einsatzes von im Spritzgussverfahren hergestellten Schläuchen kann ein zeitintensiver Arbeitsschritt bei der Herstellung einer Dispensier-Kassette ganz weggelassen werden. Zugleich ermöglicht das Verhältnis zwischen der Innen-Querschnittsfläche von 0.07 bis 7.07 mm² und einer Wanddicke von 0.3 bis 2.2 mm das vollständige Schliessen des Schlauchs durch das Spannen des Schlauchs über den Rotor. Aufgrund dieser Eigenschaft kann auf ein Schlauchbett in der Peristaltikpumpe verzichtet werden. Der Schlauch ist somit in der Lage, alleine aufgrund der Vorspannkraft zu schliessen.

Die Zahl von 33% ist das Ergebnis einer Abweichung des Innen-Durchmessers von 0.05 mm bei einer Innen-Querschnittsfläche von 0.07 mm². Somit führt eine Abweichung des Innendurchmessers von 0.05 mm bei einem Schlauch mit einem Durchmesser von 0.3 mm zu einer Veränderung der Innen-Querschnittsfläche von 33%. Je grösser der Innendurchmesser ist, umso kleiner wird die Änderung der Innen-Querschnittsfläche bei gleichbleibender Toleranz des Innendurchmessers von 0.05 mm. Bei einer Querschnittsfläche von 7.07 mm² und einem Innendurchmesser von 3 mm bewirkt die Abweichung des Innendurchmessers um 0.05 mm eine Änderung der Innen-Querschnittsfläche von 3.3%.

Die Toleranz ist so zu verstehen, dass bei einer Normalverteilung der Innen-Querschnittsflächen von erfindungsgemässen Schläuchen 95% der Schläuche eine Innen-Querschnittsfläche aufweisen, welche innerhalb des definierten Toleranzbereichs liegt. Statistisch gesehen ist die hier definierte und verwendete Toleranz zweimal die Standardabweichung. Um eine Aussage über die Toleranz der Innen-Querschnittsflächen eines Schlauchs machen zu können, muss eine statistische Auswertung durchgeführt werden, wofür eine relevante Mindestanzahl an Schläuchen zur Verfügung gestellt werden muss.

In einer bevorzugten Ausführung weist die Innen-Querschnittsfläche des Schlauchs eine Toleranz von maximal ± 13.3%, insbesondere von maximal ±6.7%, auf. Eine Toleranz von 13.3% der Innen-Querschnittsfläche korrespondiert zu einer Änderung von 0.02 mm eines Innendurchmessers von 0.3 mm. Bei einem Innendurchmesser von 3 mm bewirkt die Änderung des Innendurchmessers um 0.02 mm eine Änderung der Innen-Querschnittsfläche von 1.3%. Eine Toleranz von 6.7% der Innen-Querschnittsfläche korrespondiert wiederum zu einer Änderung von 0.01 mm des Innendurchmessers von 0.3 mm. Die Änderung um 0.01 mm eines Innendurchmessers von 3 mm führt zu einer Änderung der Innen-Querschnittsfläche von 0.7%.

In einer weiter bevorzugten Ausführung weist der Schlauch eine Innen-Querschnittsfläche von 0.502 bis 1.767 mm² und die Innen-Querschnittsfläche eine Toleranz von ± 12.5% auf. Eine Innen-Querschnittsfläche von 0.502 bis 1.767 mm² korrespondiert zu einem Innendurchmesser von 0.8 bis 1.5 mm. Eine Änderung des Innendurchmessers von 0.05 mm führt bei einem Innendurchmesser von 0.8 mm zu einer Änderung der Innen-Querschnittsfläche um 12.5%.

Vorteilhafterweise weist das Material des Schlauchs eine Shore-Härte von 20 bis 80 Shore-A, vorzugsweise von 35 bis 65 Shore-A, insbesondere bevorzugt von 45 bis 55 Shore-A, auf. Die Shore-Härte ist ein für Elastomere und Kunststoffe festgelegter und genormter Werkstoffkennwert. Für Weich-Elastomere wird der Wert in Shore-A angegeben, wobei die Skala von 0 für weich bis 100 für hart reicht. Für den Einsatz in einer Peristaltikpumpe ist die Auswahl einer dafür geeigneten Härte des Schlauch-Materials von grosser Bedeutung.

Das Material des Schlauchs weist bevorzugt eine Dichte von 0.8 bis 2.5 g/cm³, insbesondere von 1.05 bis 1.75 g/cm³, auf. Aufgrund der oben angegebenen Dimensionen und der hier vorgeschlagenen Dichte ergibt sich eine Masse für den Schlauch, welcher eine einfache Handhabung ermöglicht. Auch eine Anordnung von mehreren Schläuchen in einer Dispensier-Kassette würde weiterhin eine derart kleine Masse aufweisen, dass die Möglichkeit, die Dispensier-Kassette mit nur einer Hand zu bedienen, weiterhin gegeben wäre.

Das Material des Schlauchs umfasst vorzugsweise ein Kunststoff aus der Gruppe der Polyvynilchloride (PVC). PVC weist jene Materialeigenschaften auf, welche den Anforderungen an den Schlauch gerecht werden. Als thermoplastischer Kunststoff eignet sich PVC unter anderem ideal für das Spritzgussverfahren.

In einer weiter bevorzugten Ausführungsform umfasst das Material des Schlauchs Silikon. Silikon erfüllt ebenfalls alle Anforderungen, welche an das Material des Schlauchs gestellt werden. Darüber hinaus bietet Silikon eine bessere Umweltverträglichkeit im Vergleich zu Kunststoffen.

Wie bereits oben erwähnt, sind die Schläuche vorgesehen, in einer Dispensier-Kassette angebracht zu werden. Vorteilhafterweise sind die Verdickungen am Schlauch ausgebildet, jeweils eine formschlüssige Verbindung mit der Dispensier-Kassette einzugehen. Damit können die Schläuche mit einem minimalen Aufwand ersetzt werden. Gleichzeitig erlaubt die formschlüssige Verbindung die Schläuche jeweils immer an der gleichen Stelle zu installieren, so dass keine weitere Kontrolle der Schlauchlänge oder ähnliches notwendig bleibt. Die Verdickungen können unterschiedliche Gestalten aufweisen. Vorstellbar ist, dass die Form der Verdickungen quader-, würfel-, kugel- oder zylinderförmig gestaltet sind, um eine nicht abschliessende Liste der Formen aufgezählt zu haben. Bei einer quader- oder zylinderförmigen Verdickung verläuft die Längsachse des Quaders bzw. Zylinderachse des Zylinders stets parallel zur Längsrichtung des Schlauchs.

In einer weiter bevorzugen Ausführungsform weist der Schlauch eine Wandstärke von 0.3 mm bis 1.2 mm auf. Die Wandstärke hat einen direkten Einfluss auf den Materialaufwand. Je dünner die Wandstärke ist, umso geringer ist die erforderliche Menge des Materials, welches im Spritzgussverfahren verwendet wird. Die Wandstärke hat nebst dem Materialaufwand auch einen Einfluss auf die notwendige Quetschkraft des Schlauchs, also jene Kraft, welche aufgebracht werden muss, damit der Schlauch aufgrund seiner Verformung dicht ist. Wenn die Wandstärke stark erhöht wird, kann die erforderliche Flexibilität des Schlauchs ab einem bestimmten Punkt nicht mehr gewährleistet werden. Das wiederum hat für den Einsatz des Schlauchs in einer Peristaltikpumpe eine bedeutende Rolle.

Vorzugsweise weist der Schlauch eine Länge von 20 mm bis 70 mm auf. Die Länge des Schlauchs beeinflusst den Gesamtwiderstand auf die Flüssigkeit beim Durchströmen des Schlauchs. Um den Widerstand für die Flüssigkeit zu verkleinern kann die Länge des Schlauchs reduziert werden. Somit kann aufgrund eines kürzeren Schlauchs die gleiche Menge an Flüssigkeit mit einem kleineren Arbeitsaufwand gefördert werden.

In einer weiteren Ausführungsform können genannte Schläuche nebeneinander und miteinander verbunden angeordnet sein. Die Schläuche können parallel zueinander angeordnet sein. Vorzugsweise liegen die miteinander verbundenen Schläuche alle in einer Ebene. Die Herstellung geschieht wiederum mithilfe eines Spritzgussverfahrens. Dieses ermöglicht die Herstellung aller Schläuche inklusive der Verbindung zwischen diesen Schläuchen in einem einzigen Verfahrensschritt. Vorteilhafterweise weisen die derart miteinander verbundenen Schläuche die gleiche Länge untereinander auf.

Ein zweiter Aspekt der Erfindung betrifft eine Schlauchverbindung mit einem ersten erfindungsgemässen Schlauch und einem zweiten Schlauch. Der zweite Schlauch dient als Verlängerung des ersten Schlauchs. Vorteilhafterweise sind der erste Schlauch und der zweite Schlauch aus demselben Material. Der erste Schlauch ist weiterhin vorgesehen, um in einer Dispensier-Kassette angebracht zu werden. Der zweite Schlauch bildet dagegen die ganze oder einen Teil der Verbindung zwischen der Dispensier-Kassette und einem Behälter, in welchem die zu dispensierende Flüssigkeit aufbewahrt wird.

In einer bevorzugten Ausführung weist der erste Schlauch eine stoffschlüssige Verbindung mit dem zweiten Schlauch auf. Damit stellt sich eine feste Verbindung zwischen den Schläuchen ein, welche höheren Belastungen Stand halten kann. Durch die stoffschlüssige Verbindung findet eine eindeutige Zuordnung zwischen den beiden Schläuchen statt.

In einer weiter bevorzugten Ausführung ist der zweite Schlauch auf den ersten Schlauch aufgespritzt. Damit wird sowohl der erste als auch der zweite Schlauch in einem Spritzgussverfahren hergestellt. Dies ermöglicht die Verwendung der gleichen Materialien, was zu konstanten Materialeigenschaften über beide Schläuche hinweg führt.

In einer anderen bevorzugten Ausführung ist ein Verbindungsstück zwischen dem ersten Schlauch und dem zweiten Schlauch angeordnet und das Verbindungsstück weist eine formschlüssige Verbindung zum ersten Schlauch und zum zweiten Schlauch auf. Für die Herstellung der Verbindung zwischen dem ersten und dem zweiten Schlauch kann ein Verbindungsstück vorgesehen sein. Dies bringt den Vorteil mit sich, dass die beiden Schläuche ohne Rücksicht auf eine Verbindungsmöglichkeit mit einem anderen Schlauch produziert werden können. Die formschlüssige Verbindung des Verbindungsstücks erlaubt das beliebige Auswechseln der Schläuche.

Vorteilhafterweise ist die formschlüssige Verbindung zwischen dem Verbindungsstück und den beiden Schläuchen durch eine Steckverbindung gebildet. Die Steckverbindung bildet eine günstig herzustellende Verbindungsmöglichkeit. Dabei können die beiden zu verbinden Schläuche den gleichen Innendurchmesser aufweisen, wodurch die Geschwindigkeit der im Schlauch transportierten Flüssigkeit in beiden Schläuchen in etwa gleich bleibt.

Ein dritter Aspekt der Erfindung betrifft ein Verfahren zur Herstellung eines Schlauchs zum Anbringen an einer Dispensier-Kassette zur Aufnahme in einer Peristaltikpumpe und zum Fördern eines Mediums durch äussere mechanische Verformung des Schlauchs,
wobei der Schlauch eine Länge von vorzugsweise 20 mm bis 100 mm, eine Innen-Querschnittsfläche von 0.07 bis 7.07 mm² und eine Wandstärke von 0.3 mm bis 2.2 mm aufweist. Der Schlauch ist geeignet, um beim Spannen über den Rotor mit einer Spannkraft von 1 N bis 10 N vollständig zu schliessen. Zugleich ist der Schlauch vorgesehen, in einem Spritzgussverfahren hergestellt zu werden.

Das Herstellungsverfahren des Spritzgiessens hat den grossen Vorteil, dass die Toleranzen der Schlauch-Dimensionen viel kleiner sind als diejenigen bei zurzeit verwendeten Herstellungsverfahren.

Ein weiterer Aspekt der Erfindung betrifft die Verwendung eines erfindungsgemässen Schlauchs in einer Dispensierkassette zur Aufnahme in einer Peristaltikpumpe. Die Dosierung der Flüssigkeit wird durch das Drehen eines Rotors der Peristaltikpumpe bewirkt. Der Schlauch ist mit einer derart grossen Spannkraft über den Rotor gespannt, dass durch das alleinige Drehen des Rotors der Schlauch vollständig öffnet und schliesst, der Schlauch dabei nicht gegen eine dem Rotor gegenüberliegende Wand gedrückt wird und damit die Dosierung der Flüssigkeit stattfindet.

Noch ein weiterer Aspekt betrifft die Verwendung von mehreren erfindungsgemässen Schläuchen in einer Dispensier-Kassette zum Aufnehmen in einer Peristaltikpumpe, wobei die Schläuche parallel zueinander angeordnet und über einen Rotor der Peristaltikpumpe gespannt sind.

Genannte optionale Merkmale können in beliebiger Kombination verwirklicht werden, soweit sie sich nicht gegenseitig ausschliessen. Insbesondere dort wo bevorzugte Bereiche angegeben sind, ergeben sich weitere bevorzugte Bereiche aus Kombinationen der in den Bereichen genannten Minima und Maxima.

### KURZBESCHREIBUNG DER FIGUREN

Ausführungsbeispiele der Erfindung werden nachfolgend anhand der Figuren beispielhaft beschrieben. Es zeigen in nicht massstabsgetreuer, schematischer Darstellung:
- Figur 1:: Eine dreidimensionale Ansicht eines Schlauchs mit zwei Verdickungen;
- Figur 2:: eine dreidimensionale Ansicht eines Schlauchs mit einem kleineren Durchmesser als derjenige aus Figur 1;
- Figur 3:: eine Ansicht einer Anordnung für eine Dispensier-Kassette mit drei Kartuschen nach heutigem Stand der Technik;
- Figur 4:: eine Ansicht eines Spannelements in einer Dispensier-Kassette mit Schläuchen nach heutigem Stand der Technik;
- Figur 5:: eine Explosionsdarstellung einer Schlauchverbindung;
- Figur 6:: eine Tabelle mit der Verteilung der Innendurchmesser von erfindungsgemässen Schläuchen;
- Figur 7:: Querschnitt eines Schlauches im offenen und geschlossenen Zustand.

### DETAILIERTE BESCHREIBUNG DER FIGUREN

Im Folgenden stehen gleiche Bezugsziffern für gleiche oder funktionsgleiche Elemente (in unterschiedlichen Figuren). Ein zusätzlicher Apostroph kann zur Unterscheidung gleichartiger bzw. funktionsgleicher oder funktionsähnlicher Elemente in einer weiteren Ausführung dienen.

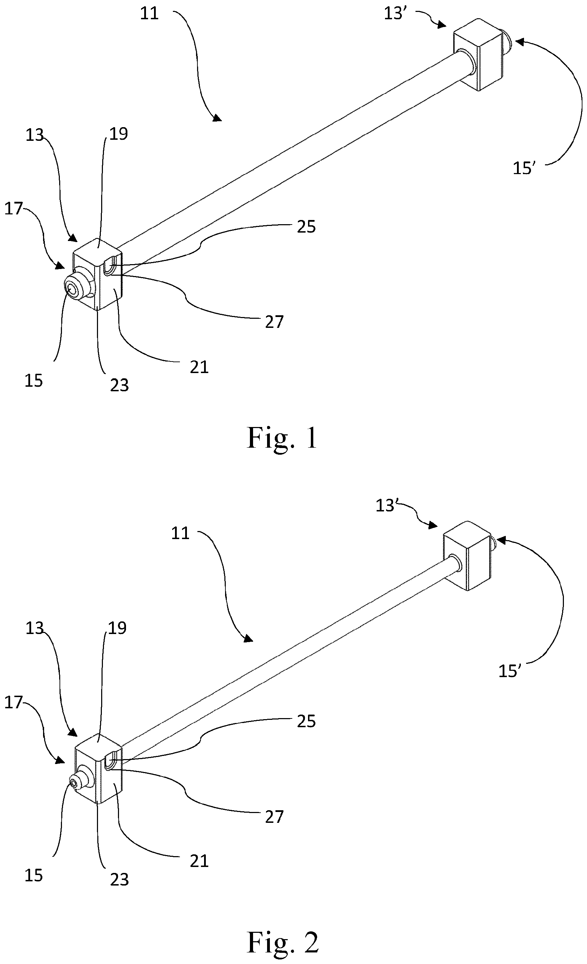

In Figur 1 ist ein Schlauch 11 gezeigt, welcher sich für das Anbringen in einer Dispensier-Kassette eignet. Die Aufgabe des Schlauchs ist das Fördern des darin enthaltenen Mediums aufgrund der eigenen mechanischen Verformung. Die erforderliche Flexibilität des Schlauchs wird idealerweise durch die Verwendung des Materials Silikon gewährleistet. Der Schlauch weist eine Länge zwischen 20 und 200 mm und einen Aussendurchmesser zwischen 2.15 mm und 3.05 mm auf. Die Toleranz des Aussendurchmesser liegt bei ± 0.02 mm.

An beiden Enden des Schlauchs ist eine quaderförmige Verdickung 13 angebracht, wobei die Verdickung nicht bis zum jeweiligen Ende 15 des Schlauchs ragt, sondern noch einen kurzen Abstand 17 zum Ende des Schlauchs aufweist. Dieser Abstand ist etwa so gross wie die Breite des Quaders 13, welcher die Verdickung bildet. Der Quader 13 weist zwei gegenüberliegende quadratische Seiten 19 und vier gleich grosse rechteckige Seiten 21 auf, wobei die rechteckigen Seiten 21 grösser sind als die quadratischen Seiten 19. Die Längskanten 23 der rechteckigen Seiten verlaufen senkrecht zur Längsrichtung des Schlauchs und sind zugleich abgerundet.

Der Schlauch weist eine Wanddicke von 0.5 mm bis 0.9 mm auf. Der Innendurchmesser beträgt zwischen 1.15 und 1.25. Die Toleranz des Innendurchmessers liegt bei ± 0.01 mm.

Auf der einen rechteckigen Seite 21 des Quaders 13 ist eine Aussparung 25 angebracht. Diese reicht von einer kurzen Kante der rechteckigen Seite bis in etwa in ihre Mitte, wo sie eine halbkreisförmige Kante 27 aufweist. Die Aussparung 25 weist in ihrer Tiefe zwei Stufen auf.

In Figur 2 ist ein Schlauch 11 gezeigt, welcher gegenüber demjenigen aus Figur 1 einen kleineren Durchmesser aufweist. Sowohl der Innendurchmesser als auch der Aussendurchmesser sind beim Schlauch 11 aus Figur 2 kleiner als diejenigen des Schlauchs aus Figur 1. Bei gleichbleibender Länge wird der Innendurchmesser beim Schlauch in Figur 2 auf 0.85 mm bis 0.95 mm reduziert. Die Toleranz des Innendurchmessers beträgt auch in dieser Ausführung ± 0.01 mm. Der Aussendurchmesser liegt zwischen 1.65 mm und 2.15 und weist eine Toleranz von ± 0.02 mm auf.

Die Verdickungen 13 des Schlauchs aus Figur 2 sind jedoch in etwa gleich gross wie diejenigen des Schlauchs aus Figur 1. Somit ergibt sich ein unterschiedliches Verhältnis zwischen den Querschnitten des Schlauchs 11 und der Verdickung 13 für die beiden Ausführungen des Schlauchs. Des Weiteren weisen die Schläuche 11 aus Figur 1 und 2 in etwa die gleiche Länge auf. Damit kommt auch ein unterschiedliches Verhältnis der Länge zum Durchmesser zwischen beiden Schläuchen zustande.

In Figur 3 ist eine Dispensier-Kassette 33 gezeigt, welche drei Kartuschen 35 umfasst. Die Kartuschen 35 weisen einen zweiteiligen Aufbau mit einem Gehäuse und einer Abdeckung auf, wobei in Figur 3 lediglich die Gehäuse der Kartuschen 35 vorliegen. In der zweiten und dritten Kartusche 35", 35‴ sind Aussparungen für die Aufnahme der Schläuche vorgesehen. Nebst diesen Aussparungen am Eintritt und Austritt der Kartusche weisen beide Kartuschen 35", 35‴ zusätzlich noch je ein Spannelement 29, 29' für jeden Schlauch 11 auf. In Figur 3 ist lediglich ein Schlauch 11 gezeigt, wobei in der Anwendung die Anzahl der Schläuche bis zu 16 betragen kann. Die Schläuche 11 werden alle nebeneinander und parallel zueinander angeordnet. In der ersten Kartusche 35' ist ein Ende des Schlauches 11 angeordnet. In der ersten Kartusche 35' übergibt der Schlauch seinen Inhalt einer Dispensier-Spitze, welche das transportiere Medium nach aussen fördert.

Nach heutigem Stand der Technik werden Schläuche 11 für die Verwendung in einer Dispensier-Kassette mit Hilfe eines Extrusion-Verfahrens hergestellt. Diese weisen eine grössere Streuung in ihrem Durchmesser auf als die erfindungsmässigen Schläuche 11. Das Resultat dieser grossen Streuung im Durchmesser der Schläuche 11 ist die Notwendigkeit, die am Schlauch angelegte Spannung in der Dispensier-Kassette individuell für jeden einzelnen Schlauch einstellen zu müssen. Zu diesem Zweck sind in den heutigen Dispenser-Kassetten Spannelemente 29 mit je einer Stellschraube 31 vorgesehen, welche in Figur 4 gezeigt sind. Die Spannung und zugleich die Länge und der Durchmesser jedes Schlauchs 11 kann über eine dafür vorgesehene Stellschraube 31 angepasst werden, so dass alle Schläuche 11 untereinander die gleiche Fördermenge aufweisen. Die erfindungsmässigen Schläuche 11 weisen aufgrund ihres Herstellungsverfahrens eine derart kleine Streuung ihres Durchmessers untereinander auf, dass zum Erreichen einer konstanten Fördermenge zwischen den Schläuchen 11 die gleiche Spannung an allen Schläuchen 11 angelegt werden kann. Dies ermöglicht es, beim Einsatz von erfindungsmässigen Schläuchen 11 auf die Verwendung von Spannelementen 29 und Stellschrauben 31 in der Dispensier-Kassette zu verzichten.

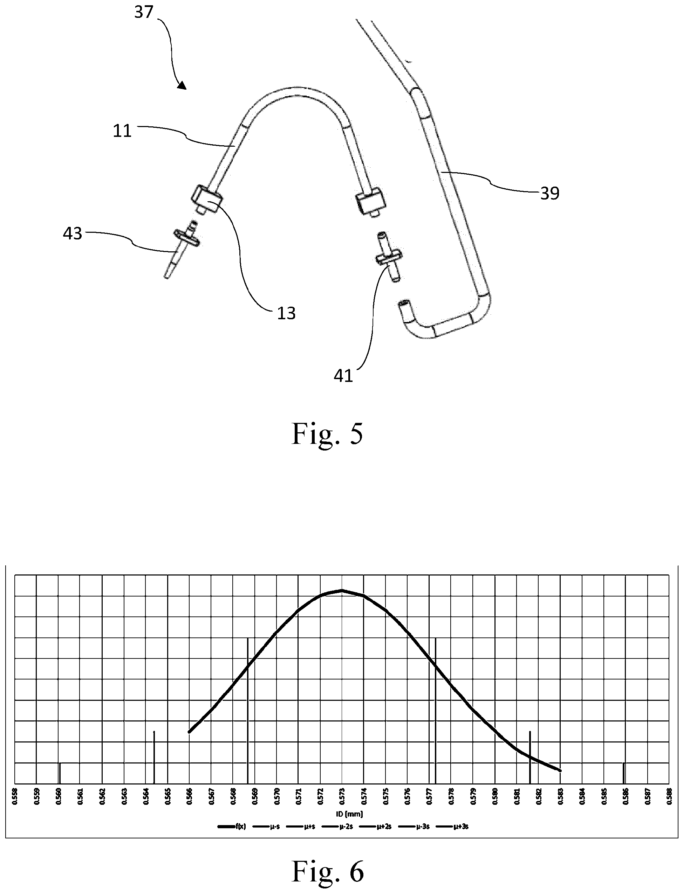

In Figur 5 ist eine Schlauchverbindung 37 aus einem erfindungsmässigen ersten Schlauch 11 und einem zweiten Schlauch 39 dargestellt. Zwischen dem ersten 11 und dem zweiten Schlauch 39 ist ein Verbindungsstück angeordnet 41. Dieses umfasst einen kurzen Rohrabschnitt, dessen Aussenwände an beiden Enden einen konischen Verlauf aufweisen. Das Verbindungsstück 41 ist vorgesehen, mit beiden Enden in je einen der zu verbindenden Schläuche 11,37 eingeführt zu werden. Durch Überziehen des Schlauches über das Verbindungsstück 41 stellt sich aufgrund des konischen Verlaufs der Aussenwand des Verbindungsstücks und der Elastizität des Schlauches eine Reibschluss-Verbindung ein. Am anderen Ende des ersten Schlauchs 11 kann eine Düse 43 angebracht werden. Diese weist wie das Verbindungsstück 41 ebenfalls eine konische Aussenwand auf, damit sie auch in den Schlauch 11 eingeschoben werden kann. An dem vom Schlauch 11 gegenüberliegenden Ende der Düse 43 ist eine Verengung sowohl des Aussen- als auch des Innendurchmessers vorgesehen. Damit wird die Querschnittsfläche reduziert, was beim Dispensieren das Nachtropfen der Flüssigkeit hemmt.

Die Figur 6 zeigt eine Tabelle mit der Streuung der Innendurchmesser von verschiedenen erfindungsgemässen Schläuchen. Die Verteilung der Innendurchmesser zeigt erwartungsgemäss eine Normalverteilung mit dem mittleren Innendurchmesser von 0.573 mm. Die Normalverteilung erlaubt eine Aussage über die Definition der Toleranz zu machen. Bei einer Messreihe mit einer Normalverteilung stellt die Toleranz jenen Wertebereich dar, in welchem 95% der gemessenen Werte enthalten sind. In diesem Fall liegen 95% der gemessenen Innendurchmesser in einem Bereich von 0.573 ± 0.0172 mm, womit die Toleranz des Durchmessers als ± 0.0172 mm bestimmt ist. Der hier gewählte Durchmesser von 0.573 mm bildet lediglich einen von vielen möglichen Innendurchmesser des Schlauchs im Bereich zwischen 0.3 mm bis 3.0 mm. Unabhängig von der Wahl des Soll-Durchmessers ergibt die Verteilung der Durchmesser einer Mehrzahl von Schläuchen eine Normalverteilung nach Gauss um den gewählten Soll-Durchmesser.

Für die Messung der Innen-Querschnittsfläche muss die Form der Querschnittsfläche bestimmt werden. Falls der Schlauch einen kreisförmigen Innen-Querschnitt aufweist, kann vom Innendurchmesser auf die Innen-Querschnittsfläche geschlossen werden. Falls der Querschnitt eine rechteckige oder quadratische Form aufweist, müssen die Seitenlängen gemessen werden, um die Innen-Querschnittsfläche zu berechnen. Da die meisten Schläuche einen kreisförmigen Querschnitt aufweisen, wird das Messverfahren zur Bestimmung der Innen-Querschnittsfläche im Folgenden anhand eines runden Schlauch-Querschnitts beschrieben.

Für das Bestimmen der Innen-Querschnittsfläche sind zwei Methoden bekannt. Beide Methoden verfügen über eine derart hohe Messgenauigkeit, dass die Verteilung der gemessenen Werte auf die Herstellungstoleranz zurückschliessen lässt.

Die erste Methode betrifft die Messung des Innendurchmesser eines Schlauchs mit Hilfe eines Computertomographen. Die Messgenauigkeit des Computertomographen liegt bei ungefähr 30 µm. Für die Bestimmung der Toleranz der Innen-Querschnittsfläche müssen die Innen-Durchmesser gemessen werden. Von diesen kann anschliessend auf die Innen-Querschnittsflächen geschlossen werden. Die zu erwartende Verteilung ist eine Gauss- oder Normal-Verteilung. Diese geht davon aus, dass sich die Verteilung der gemessenen Innen-Durchmesser um den Sollwert symmetrisch in beide Richtungen erstreckt. Dabei definieren die Grenzwerte, welche den Bereich abdecken, in welchem 95% der gemessen Innen-Durchmesser zu liegen kommen, die Toleranzwerte des Innen-durchmessers. Die Messung ist an mindestens zwei Punkten des Schlauchs durchzuführen. Vorzugsweise liegen diese Punkte nicht nahe beieinander. Am geeignetsten ist die Wahl eines ersten Messpunktes nahe an einem Ende des Schlauchs und die Wahl des zweiten Messpunktes in der Nähe des anderen Endes des Schlauchs.

Eine zweite Methode zur Bestimmung der Innen-Durchmesser bildet die Messung durch Durchströmen der Schläuche mit gleichen und bekannten Randbedingungen. Von den Randbedingungen kann auf den Volumenstrom geschlossen werden. Somit kann der sich einstellende Volumenstrom mit dem Soll-Volumenstrom verglichen werden. Die Differenz dieser Volumenströme ist auf eine unterschiedliche Innen-Querschnittsfläche zurückzuführen. Der Volumenstrom durch einen Schlauch verhält sich proportional zu seiner Innen-Querschnittsfläche. Diese Messmethode bedingt, dass entweder ein Schlauch, von welchem die Genauigkeit der Dimensionen bereits bekannt ist, als Referenzgrösse oder ein zu erreichender Volumenstrom als Vergleichsgrösse verwendet wird. Der Referenzschlauch definiert den Soll-Volumenstrom. Die Abweichung der gemessenen Volumenströme der übrigen Schläuche vom Soll-Volumenstrom ist auf die unterschiedliche Innen-Querschnittsfläche der übrigen Schläuche gegenüber dem Referenzschlauch zurückzuführen. Da wie oben erwähnt der Volumenstrom proportional zur Innen-Querschnittsfläche ist, kann von der Differenz des Volumenstroms direkt auf die Differenz der Innen-Querschnittsfläche geschlossen werden. Falls lediglich eine Vergleichsgrösse bekannt ist, muss jeder gemessene Volumenstrom mit der Vergleichsgrösse verglichen werden. Die Abweichung zur Vergleichsgrösse bestimmt die Abweichung der Innen-Querschnittsfläche von der Soll-Innen-Querschnittsfläche. Dabei verhält sich die Diskrepanz im Volumenstrom proportional zu derjenigen in der Innen-Querschnittsfläche.

Der Volumenstrom hier muss nicht als ein Volumen in Abhängigkeit einer Zeiteinheit beschrieben sein, sondern kann anstelle der Zeit auch andere Masseinheiten verwenden. Diese Masseinheiten können auch zeitunabhängig sein wie zum Beispiel die Rotorumdrehung einer Peristaltikpumpe. Somit kann der Volumenstrom als Volumen pro Rotorumdrehung der Peristaltikpumpe sowohl als Vergleichsgrösse als auch als Messgrösse angegeben sein. Für die Bestimmung, ob ein Schlauch innerhalb des Toleranzbereichs liegt oder nicht, muss eine grössere Menge von Schläuchen der gleichen Ausführung gemessen werden. Wenn mindestens 95% aller gemessener Schläuche einen Wert innerhalb des Toleranzbereichs aufweisen, so gilt der Schlauch als innerhalb des Toleranzbereichs liegend. Es ist nicht möglich, aufgrund einer einzelnen Messung eines Schlauchs eine Aussage zu treffen, ob dieser Schlauch im Allgemeinen innerhalb eines Toleranzbereichs liegt oder nicht.

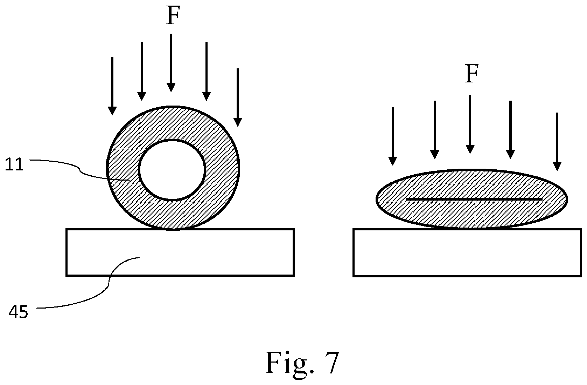

In Figur 7 ist eine Abfolge von zwei Darstellungen gezeigt, welche das Schliessen eines Schlauchs 11 zeigen. Der Schlauch ist dabei auf einem Steg 45 eines Rotor angeordnet. Die auf den Schlauch 11 wirkende Kraft F ist ein Ergebnis der Vorspannkraft, mit welcher der Schlauch 11 in der Dispensier-Kassette gespannt ist, und der Gegenkraft des Steges 45, welcher mit der Bewegung des Rotors sich verändert. Während die Vorspannkraft konstant bleibt, variiert die Gegenkraft des Steges in Abhängigkeit von dessen Position. Somit kommt es durch das Drehen des Rotors zu einer zyklischen Öffnungs- und Schliessbewegung des Schlauchs 11.

Die Amplitude der durch den Steg 45 ausgeübten Gegenkraft kann nicht verändert werden. Die Vorspannkraft, mit welcher der Schlauch 11 an der Dispensier-Kassette gespannt ist, kann dagegen beliebig eingestellt werden. Mit der Vorspannkraft kann die Lebensdauer des Schlauchs 11 beeinflusst werden. Je grösser die Vorspannkraft umso kürzer ist die Lebensdauer des Schlauchs 11.

Die Auswahl der Vorspannkraft ist des Weiteren von der Geometrie des Schlauchs 11 insbesondere von dessen Querschnittsfläche abhängig. Je grösser die Querschnittsfläche des Schlauchs 11 ist, umso grösser muss die Vorspannkraft sein, damit der Schlauch vollständig schliesst.

Ein weiterer Effekt, der bei der Dimensionierung des Schlauchs 11 zu berücksichtigen ist, ist die Trägheit und der Druck des im Schlauch 11 sich befindenden Flüssigkeit. Wenn die auf den Schlauch 11 ausgeübte Kraft F durch die Vorspannung einen bestimmten Wert unterschreitet, schliesst der Schlauch 11 nicht mehr vollständig, da der innere Druck im Schlauch 11 eine grössere Kraft ausübt.

Der Schlauch 11 muss mindestens einen bestimmten Durchmesser aufweisen, damit das vollständige Schliessen überhaupt gewährleistet ist. Wird dieser Wert unterschritten, führt die alleinige Kraft durch den Druck der Flüssigkeit im Schlauch zu einer ständigen Teil-Öffnung des Schlauchs.

Unter Berücksichtigung dieser Verhaltensweisen und Regeln ergibt sich ein Bereich der Querschnittsfläche, in welchem die Schliessung des Schlauchs gewährleistet ist und eine maximale Lebensdauer des Schlauches 11 erzielt wird.

Versuche und Messungen haben gezeigt, dass bei einer Vorspannkraft von 1 bis 10 N die optimale Lebensdauer für ein Schlauch mit einem Innendurchmesser von etwa 0.9 mm durch eine Wanddicke von etwa 0.5 mm erzielt wird. Je grösser die Wanddicke ist, umso grösser muss die Vorspannkraft sein. Da die maximale Lebensdauer mit der kleinsten Vorspannkraft erreicht wird, sollte für die maximale Lebensdauer die möglichst kleinste Wanddicke gewählt werden. Somit ergibt sich zum Erzielen einer maximalen Lebensdauer bei einem Schlauch-Innendurchmesser von 0.15 mm bis 1.5 mm eine ideale Wanddicke von 0.3 bis 2.2 mm, insbesondere von 0.3 bis 1.2 mm, weiter bevorzugt von 0.3 bis 0.7 mm.

Ein kleiner Innen-Durchmesser bedingt auch eine kleinere Wanddicke. Deshalb wird für ein Schlauch, welcher beim Spannen über ein Rotor mit einer Zugkraft von 1 bis 10 N schliesst, das Verhältnis zwischen dessen Innen- und Aussendurchmesser angegeben. Idealerweise ist das Verhältnis zwischen dem Innen- und Aussendurchmesser des Schlauchs 0.3 bis 0.6, vorzugsweise 0.4 bis 0.55.

### Verwendung eines erfindungsgemässen Schlauchs

Ein erfindungsgemässer Schlauch ist vorgesehen, über dessen Verdickungen an einer Dispensierkassette angebracht zu sein. Bevorzugt werden in der Dispensierkassette mehrere Schläuche parallel angeordnet. Die Herstellung der Schläuche durch das Spritzguss-Verfahren führt zu sehr kleinen Toleranzen des Innendurchmessers. Aufgrund der kleinen Toleranz brauchen die einzelnen Schläuche nicht individuell eingestellt zu werden. Zugleich wird damit auch ermöglicht, einen einzelnen Schlauch durch einen anderen zu ersetzen, ohne eine Änderung an der Dispensierkassette vornehmen zu müssen. Der erfindungsgemässe Schlauch ist derart dimensioniert und an der Dispensierkassette angebracht, dass der Schlauch beim Betrieb in einer Peristaltikpumpe mit nur einem Rotor und ohne Schlauchbett vollständig schliessen kann. Der Schlauch ist während dem Betrieb in der Peristaltikpumpe über den Rotor gespannt. Die Drehung des Rotors ist für die Peristaltik verantwortlich. Dabei wird der erfindungsgemässe Schlauch vollständig geschlossen, ohne dafür auf eine Gegenkraft in Form eines Schlauchbetts angewiesen zu sein. Die vollständige Schliessung des Schlauchs wird alleine durch die Kraft zum Quetschen des Schlauchs ermöglicht. Diese wird durch die Spannkraft des Schlauchs über den Rotor, die Rotorgeometrie und der Dimension des Schlauchs bestimmt. Der Schlauch muss eine derart hohe Wanddicke aufweisen, dass die Masse der Wand für eine genügend grosse Kraft zum Klemmen des Schlauches und somit zur vollständigen Schliessung sorgt. Wie bereits oben beschrieben führen eine zu grosse Spannkraft oder eine zu dünne Wanddicke des Schlauchs zu einer Reduktion der Lebensdauer des Schlauchs.

Während die Erfindung vorstehend unter Bezugnahme auf spezifische Ausführungsformen beschrieben wurde, ist es offensichtlich, dass Änderungen, Modifikationen, Variationen und Kombinationen ohne vom Erfindungsgedanken abzuweichen gemacht werden können. Der Umfang der Erfindung ist in den beigefügten Ansprüchen definiert.

### BEZUGSZEICHENLISTE:

- 11: Schlauch
- 13, 13': Verdickung am Schlauch
- 15, 15': Ende des Schlauchs
- 17: Abstand von der Verdickung bis zum Ende des Schlauchs
- 19: Quadratische Seite einer quaderförmigen Verdickung
- 21: Rechteckige Seite einer quaderförmigen Verdickung
- 23: Längskanten der Verdickung
- 25: Aussparung an der Verdickung
- 27: Halbkreisförmige Kante der Verdickung
- 29: Spannelemente
- 31: Stellschraube
- 33: Dispensier-Kassette
- 35, 35', 35", 35‴: Kartusche
- 37: Schlauchverbindung
- 39: Zweiter Schlauch
- 41: Verbindungsstück
- 43: Düse
- 45: Steg des Rotors

## Patentansprüche

1. Schlauch (11) zum Anbringen an einer Dispensier-Kassette (33) zur Aufnahme in einer Peristaltikpumpe und zum Spannen über einen Rotor der Peristaltikpumpe und Fördern eines Mediums durch äussere mechanische Verformung des Schlauchs,
wobei der Schlauch (11) in einem Spritzgussverfahren hergestellt ist und eine Länge von vorzugsweise 20 mm bis 200 mm, aufweist, und
Verdickungen (13) an beiden Enden des Schlauchs (11) angeformt sind und die Distanz zwischen den Verdickungen zwischen 40 mm und 100 mm beträgt,
**dadurch gekennzeichnet,**
**dass** der Schlauch (11) eine Innen-Querschnittsfläche von 0.07 bis 7.07 mm² und eine Wandstärke von 0.3 mm bis 2.2 mm, vorzugsweise von 0.3 bis 1.5mm, aufweist,,
die Innen-Querschnittsfläche eine Toleranz von maximal ± 33% aufweist und der Schlauch (11) geeignet ist, um beim Spannen über den Rotor mit einer Spannkraft von 1 bis 10 N vollständig zu schliessen.

2. Schlauch (11) nach Anspruch 1, **dadurch gekennzeichnet, dass** die Innen-Querschnittsfläche des Schlauchs (11) eine Toleranz von maximal ± 13.3%, insbesondere von maximal ± 6.7% aufweist.

3. Schlauch (11) nach Anspruch 1 oder 2, **dadurch gekennzeichnet, dass** der Schlauch (11) eine Innen-Querschnittsfläche von 0.502 bis 2.6 mm², insbesondere von 0.502 bis 1.767 mm², und die Innen-Querschnittsfläche eine Toleranz von maximal ± 12.5% aufweist.

4. Schlauch (11) nach einem der Ansprüche 1 bis 3, **dadurch gekennzeichnet, dass** das Material des Schlauchs eine Shore-Härte von 20 bis 80 Shore-A, vorzugsweise von 35 bis 65 Shore-A, insbesondere bevorzugt von 45 bis 55 Shore-A, aufweist.

5. Schlauch (11) nach einem der Ansprüche 1 bis 4, **dadurch gekennzeichnet, dass** das Material des Schlauchs ein Kunststoff aus der Gruppe der Polyvynilchloride (PVC) umfasst.

6. Schlauch (11) nach einem der Ansprüche 1 bis 4, **dadurch gekennzeichnet, dass** das Material des Schlauchs Silikon umfasst.

7. Schlauch (11) nach einem der Ansprüche 1 bis 6, **dadurch gekennzeichnet, dass** die Verdickungen (13) ausgebildet sind, jeweils eine formschlüssige Verbindung mit der Dispensier-Kassette (33) einzugehen.

8. Schlauch (11) nach einem der Ansprüche 1 bis 7, **dadurch gekennzeichnet, dass** der Schlauch (11) eine Wandstärke von 0.3 mm bis 1.2 mm aufweist.

9. Schlauch (11) nach einem der Ansprüche 1 bis 8, **dadurch gekennzeichnet, dass** der Schlauch (11) eine Länge von 20 mm bis 70 mm aufweist.

10. Schlauchverbindung (37) mit einem ersten Schlauch (11) nach einem der Ansprüche 1 bis 9 und einem zweiten Schlauch (39).

11. Schlauchverbindung (37) nach Anspruch 11, **dadurch gekennzeichnet, dass** der erste Schlauch (11) eine stoffschlüssige Verbindung mit dem zweiten Schlauch (39) aufweist.

12. Schlauchverbindung (37) nach Anspruch 11 oder 12, **dadurch gekennzeichnet, dass** der erste Schlauch (11) auf den zweiten Schlauch (39) aufgespritzt ist.

13. Schlauchverbindung (37) nach Anspruch 11, **dadurch gekennzeichnet, dass** ein Verbindungsstück (41) zwischen dem ersten Schlauch (11) und dem zweiten Schlauch (39) angeordnet ist und das Verbindungsstück (41) eine formschlüssige Verbindung zum ersten Schlauch (11) und zum zweiten Schlauch (39) aufweist.

14. Verfahren zur Herstellung eines Schlauchs (11) gemäss einem der Ansprüche 1 bis 9,
**dadurch gekennzeichnet,**
**dass** der Schlauch (11) in einem Spritzgussverfahren hergestellt wird.

15. Verwendung eines Schlauches gemäss den Ansprüchen 1 bis 9 zum Anbringen in einer Dispensierkassette zur Aufnahme in einer Peristaltikpumpe, wobei die Dosierung der Flüssigkeit durch das Drehen eines Rotors der Peristaltikpumpe bewirkt wird und der Schlauch mit einer derart grossen Spannkraft über den Rotor gespannt ist, dass durch das alleinige Drehen des Rotors der Schlauch vollständig öffnet und schliesst,
**dadurch gekennzeichnet, dass** der Rotor zur Dosierung den Schlauch nicht gegen eine Wand drückt.

16. Verwendung von mehreren Schläuchen gemäss einem der Ansprüche 1 bis 9 in einer Dispensier-Kassette zum Aufnehmen in einer Peristaltikpumpe, wobei die Schläuche (11) parallel zueinander angeordnet und über einen Rotor der Peristaltikpumpe gespannt sind.

## Claims

1. A hose (11) for attachment to a dispensing cartridge (33) for placement in a peristaltic pump and for stretching over a rotor of the peristaltic pump and conveying a medium by external mechanical deformation of the hose,
said hose (11) being produced by an injection molding process and having a length of preferably from 20 mm to 200 mm, and
thickenings (13) being formed at both ends of the hose (11), whereby the distance between the thickenings is between 40 mm and 100 mm,
**characterized in that**
the hose has an internal cross-sectional area of from 0.07 to 7.07 mm² and a wall thickness of from 0.3 mm to 2.2 mm, preferably from 0.3 to 1.5 mm,
the internal cross-sectional area has a tolerance of not more than ± 33% and the hose (11) is suitable for closing completely when stretched over the rotor with a tension force of 1 to 10 N.

2. Hose (11) in accordance with claim 1, **characterized in that** the inner cross-sectional area of the hose (11) has a tolerance of at most ± 13.3%, in particular at most ± 6.7%.

3. Hose (11) in accordance with claim 1 or 2, **characterized in that** the hose (11) has an inner cross-sectional area of 0.502 to 2.6 mm², in particular of 0.502 to 1.767 mm², and the inner cross-sectional area has a tolerance of at most ± 12.5%.

4. Hose (11) in accordance with claim 1, **characterized in that** the material of the hose has a Shore hardness of from 20 to 80 Shore-A, preferably from 35 to 65 Shore-A, more preferably from 45 to 55 Shore-A.

5. Hose (11) in accordance with claim 1, **characterized in that** the material of the hose comprises a plastic selected from the group consisting of polyvinyl chlorides (PVC).

6. Hose (11) in accordance with claim 1, **characterized in that** the material of the hose comprises silicone.

7. Hose (11) in accordance with claim 1, **characterized in that** the thickenings (13) are each configured to form a positive connection with the dispensing cartridge (33).

8. Hose (11) in accordance with claim 1, **characterized in that** the hose (11) has a wall thick-ness of 0.3 mm to 1.2 mm.

9. Hose (11) in accordance with claim 1, **characterized in that** the hose (11) has a length of 20 mm to 70 mm.

10. Hose connection (37) comprising a first hose (11) in accordance with claim 1 and a second hose (39).

11. Hose connection (37) in accordance with claim 10, **characterized in that** the first hose (11) has a material connection with the second hose (39).

12. The hose connection (37) in accordance with claim 10 or 11, **characterized in that** the first hose (11) is overmolded onto the second hose (39).

13. A hose connection (37) in accordance with claim 10, **characterized in that** a connecting piece (41) is arranged between the first hose (11) and the second hose (39) and the connecting piece (41) has a positive connection to the first hose (11) and to the second hose (39).

14. A method of manufacturing a hose (11) in accordance with any of claims 1 to 9, **characterized in that** the hose (11) is produced by an injection molding process.

15. Use of a hose in accordance with any of claims 1 to 9 for mounting in a dispensing cartridge for placement in a peristaltic pump, wherein the dosing of the liquid is effected by the rotation of a rotor of the peristaltic pump and the hose is stretched over the rotor with tension force sufficient for the hose to open and close solely by the rotation of the rotor, **characterized in that** the rotor for dosing does not press the hose against a wall.

16. Use of a plurality of hoses in accordance with claim 1 in a dispensing cartridge for placement in a peristaltic pump, whereby the hoses (11) are arranged parallel to each other and stretched over a rotor of the peristaltic pump.

## Revendications

1. Tuyau (11) à fixer à une cassette de distribution (33) pour être logé dans une pompe péristaltique et pour être serré par un rotor de la pompe péristaltique et pour transporter un milieu par déformation mécanique extérieure du tuyau,
cependant que le tuyau (11) est fabriqué par moulage par injection et présente une longueur de préférence de 20 mm à 200 mm et que des renflements (13) sont moulés aux deux extrémités du tuyau (11) et la distance entre les renflements est entre 40 mm et 100 mm,
**caractérisé en ce**
**que** le tuyau (11) présente une surface de section interne de 0,07 à 7,07 mm² et une épaisseur de paroi de 0,3 mm à 2,2mm, de préférence de 0,3 à 1,5 mm,
la surface de section interne présente une tolérance de ± 33% maximum et le tuyau (11) est approprié pour se fermer complètement lors du serrage par le rotor avec une force de serrage de 1 à 10 N.

2. Tuyau (11) selon la revendication 1, **caractérisé en ce que** la surface de section interne du tuyau (11) présente une tolérance de ± 13,3%, en particulier de ± 6,7% maximum.

3. Tuyau (11) selon la revendication 1 ou 2, **caractérisé en ce que** le tuyau présente une surface de section interne de 0,502 à 2,6 mm², en particulier de 0,502 à 1,767 mm² et la surface de section interne présente une tolérance de ± 12,5% maximum.

4. Tuyau (11) selon l'une des revendications 1 à 3, **caractérisé en ce que** le matériau du tuyau présente une dureté Shore de 20 à 80 Shore-A, de préférence de 35 à 65 Shore-A, en particulier de manière préférée de 45 à 55 Shore-A.

5. Tuyau (11) selon l'une des revendications 1 à 4, **caractérisé en ce que** le matériau du tuyau comprend une matière synthétique des polychlorures de vinyle (PVC).

6. Tuyau (11) selon l'une des revendications 1 à 4, **caractérisé en ce que** le matériau du tuyau comprend du silicone.

7. Tuyau (11) selon l'une des revendications 1 à 6, **caractérisé en ce que** les renflements (13) sont configurés pour réaliser respectivement un assemblage par conjugaison de forme avec la cassette de distribution (33).

8. Tuyau (11) selon l'une des revendications 1 à 7, **caractérisé en ce que** le tuyau (11) présente une épaisseur de paroi de 0,3 à 1,2 mm.

9. Tuyau (11) selon l'une des revendications 1 à 8, **caractérisé en ce que** le tuyau (11) présente une longueur de 20 mm à 70 mm.

10. Assemblage de tuyau (37) avec un premier tuyau (11) selon l'une des revendications 1 à 9 et un second tuyau (39).

11. Assemblage de tuyau (37) selon la revendication 11, **caractérisé en ce que** le premier tuyau 11) présente un assemblage par alliance de matière avec le second tuyau (39).

12. Assemblage de tuyau (37) selon la revendication 11 ou 12, **caractérisé en ce que** le premier tuyau (11) est surmoulé sur le second tuyau (39).

13. Assemblage de tuyau (37) selon la revendication 11, **caractérisé en ce qu'**une pièce de jonction (41) est placée entre le premier tuyau (11) et le second tuyau (39) et la pièce de jonction (41) présente un assemblage par conjugaison de forme avec le premier tuyau (11) et le second tuyau (39).

14. Procédé pour la fabrication d'un tuyau (11) selon l'une des revendications 1 à 9, **caractérisé en ce**
**que** le tuyau (11) est fabriqué par moulage par injection.

15. Utilisation d'un tuyau (11) selon l'une des revendications 1 à 9 à fixer à une cassette de distribution (33) pour être logé dans une pompe péristaltique, cependant que le dosage du liquide est causé par la rotation d'un rotor de la pompe péristaltique et le tuyau est serré par le rotor avec une force de serrage telle que le tuyau s'ouvre et se ferme complètement par la seule rotation du rotor,
**caractérisée en ce que** le rotor pour le dosage ne presse pas le tuyau contre une paroi.

16. Utilisation de plusieurs tuyaux selon l'une des revendications 1 à 9 dans une cassette de distribution pour être logés dans une pompe péristaltique, cependant que les tuyaux (11) sont placés parallèlement l'un à l'autre et sont serrés par un rotor de la pompe péristaltique.
